# EUROPÄISCHE PATENTANMELDUNG

(11) **EP 2 014 287 A1**
(43) Veröffentlichungstag der Anmeldung: **14.01.2009**
(21) Anmeldenummer: 07090117.8
(22) Anmeldetag: 13.06.2007
(51) Int. Cl.: A61K 31/4045, A61K 31/4439, C07D 209/14, C07D 401/12, A61P 15/00

(54) **Verwendung von Zimtsäurederivaten als Modulatoren des EP2-Rezeptors**

(71) Anmelder: Bayer Schering Pharma Aktiengesellschaft, 13353 Berlin (DE)
(72) Erfinder: Buchmann, Bernd, 16540 Hohen Neuendorf (DE); Bräeuer, Nico, 07743 Jena (DE); Koppitz, Marcus, 10115 Berlin (DE); Peters, Olaf, 99891 Tabarz (DE); Eis, Knut, 13587 Berlin (DE); Terlaak, Antonius, 12159 Berlin (DE); Lindenthal, Bernhard, 13507 Berlin (DE); Langer, Gernot, 14612 Falkensee (DE); Wintermantel, Tim, 10178 Berlin (DE)

(57) **Zusammenfassung**

Die vorliegende Erfindung betrifft die Verwendung von Zimtsäurederivaten der allgemeinen Formel I zur Herstellung von pharmazeutischen Mitteln zur Behandlung von Erkrankungen und Indikationen, die im Zusammenhang stehen mit dem EP₂ Rezeptor sowie Verfahren zu ihrer Herstellung.

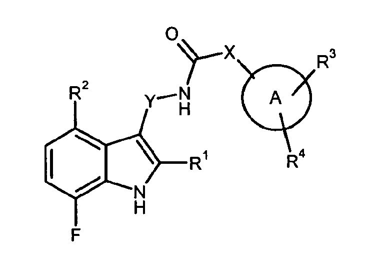

## Beschreibung

Die vorliegende Erfindung betrifft die Verwendung von Zimtsäurederivaten als EP₂ Rezeptor Modulatoren und deren Verwendung als Arzneimittel sowie Verfahren zu ihrer Herstellung.

Es ist schon lange bekannt, dass Prostaglandine Schlüsselmoleküle bei den Prozessen der weiblichen Reproduktionsbiologie, wie z. B. der Regulation der Ovulation, der Fertilisation, der Nidation, der Dezidualisierung (z.B. Plazentabildung) und der Menstruation, sind. Prostaglandine spielen ebenfalls eine wichtige Rolle bei den pathologischen Veränderungen im reproduktiven Trakt, einschließlich der Menorrhagie, Dysmenorrhoe, Endometriose und Krebs. Bisher ist der Mechanismus, durch den Prostaglandine diese Veränderungen bewirken, noch nicht vollständig geklärt. Neuere Erkenntnisse weisen darauf hin, dass Prostaglandine, ihre Rezeptoren und deren Signaltransduktionswege an Prozessen wie Angiogenese, Apoptose, Proliferation sowie an inflammatorischen/antiinflammatorischen und immunologischen Prozessen beteiligt sind.

Die Wirkungen der Prostaglandine werden durch ihre G-Protein-gekoppelten Rezeptoren, die auf der Zelloberfläche lokalisiert sind, vermittelt. Von besonderem Interesse ist das Prostaglandin E₂ (PGE₂), das unterschiedlichste zelluläre Wirkungen erzielt, indem es an funktionell verschiedene Rezeptorensubtypen, nämlich die EP₁, EP₂, EP₃ und EP₄ Rezeptoren, bindet. Die Rezeptor-Subtypen, an denen das Prostaglandin E₂ bindet, scheinen für die Rezeptor vermittelten Wirkungen, die bei der Fertilitätsregulation eine Rolle spielen, von besonderem Interesse zu sein. So konnte gezeigt werden, dass die reproduktiven Funktionen bei EP₂ Knock-out Mäusen (EP₂ ^{-/-}), d.h. bei Mäusen, die keinen funktionstüchtigen PGE₂-Rezeptor Subtyp EP₂ mehr besitzen, beeinträchtigt sind, und dass diese Tiere eine kleinere "Wurfanzahl" haben (Matsumoto et al., 2001, Biology of Reproduction 64, 1557-1565). Ebenso konnte gezeigt werden, dass diese EP₂ Knock-out Mäuse (Hizaki et al. Proc Natl Acad Sci U. S. A. 1999 Aug 31; 96(18):10501-10506) eine deutlich verminderte Kumulus Expansion und starke Subfertilität aufweisen, was in ursächlichem Zusammenhang mit verminderten Reproduktionsprozessen, wie der Ovulation und Fertilisierung, zu sehen ist.

Der EP₂-Rezeptor stellt demnach ein wichtiges Ziel für die Entwicklung von Arzneimitteln für die Regulation der weiblichen Fertilität dar. Die Existenz der 4 Subklassen des PGE₂-Rezeptors eröffnet die Möglichkeit zur gezielten Entwicklung selektiv wirksamer Verbindungen. Bisher sind allerdings kaum selektive EP₂-Rezeptor-Liganden bekannt, die an den EP₂-Subtypen des PGE₂-Rezeptors binden, da die meisten bekannten Verbindungen auch an die anderen PGE₂-Rezeptor-Subtypen, wie beispielsweise an den EP₄-Rezeptor, binden.

EP₂-Rezeptor Antagonisten werden beispielsweise in der Anmeldung US2005059742 beschrieben (Jabbour, Medical Research Concil). Beansprucht wird eine Methode, bei der ein EP₂ und/oder ein EP₄ Antagonist zur Behandlung von Menorrhagie und Dysmenorrhoe eingesetzt werden kann. AH6809 wird als Antagonist des EP₂ oder EP₄ Rezeptors offenbart, es werden keine anderen spezifischen Antagonisten und keine neuen Verbindungen offenbart.

In einer früheren Anmeldung derselben Gruppe (EP1467738) werden EP₂ oder EP₄ Antagonisten zur Behandlung von pathologischen Zuständen, wie z.B. allergische Erkrankungen, Morbus Alzheimer, Schmerz, Abort, Menstruationsbeschwerden, Menorrhagie und Dysmenorrhoe, Endometriose, Erkrankungen der Knochen, Ischämie usw. Die beschriebenen Verbindungen zeichnen sich jedoch durch eine besonders hohe Affinität zum EP₃ Rezeptor aus. In einer weiteren Anmeldung (W004/032964) werden neue Verbindungen beschrieben, die sich ebenfalls durch eine besonders hohe Affinität zum EP₃ Rezeptor auszeichnen, aber auch EP₂ antagonistisch wirken und die für Behandlung und Prophylaxe von allergischen Erkrankungen Verwendung finden.

Ono Pharmaceutical beansprucht in der Anmeldung W003/016254 die Herstellung von Benzolsäure oder gesättigten Carbonsäure-Derivaten, die mit Aryl oder Heterocyclen substituiert sind, unter anderem als PGE₂-Rezeptor Antagonisten. Die offenbarten Verbindungen werden für die Behandlung einer Vielzahl von Erkrankungen beansprucht, darunter auch allergische Erkrankungen, Morbus Alzheimer, Schmerz, Abort, Menstruationsbeschwerden, Menorrhagie und Dysmenorrhoe, Endometriose, Erkrankungen der Knochen, Ischämie usw. Die beschriebenen Verbindungen zeichnen sich jedoch durch eine besonders hohe Affinität zum EP₃ Rezeptor aus. In einer weiteren Anmeldung (W004/032964) werden neue Verbindungen beschrieben, die sich ebenfalls durch eine besonders hohe Affinität zum EP₃ Rezeptor auszeichnen, aber auch als EP₂ Antagonisten, für die Behandlung und Prophylaxe von allergischen Erkrankungen, Verwendung finden.

In der Anmeldung W004/39807 der Firma Merck Frosst, Canada, wird die Herstellung von Pyridopyrrolizinen und Pyridoindolizinen offenbart. Diese Verbindungen zeichnen sich jedoch durch gute Bindung an den PGD₂ Rezeptor aus, dieser Rezeptor stellt einen anderen Subtyp des Prostaglandinrezeptors dar.
Naphathalinderivate als EP₄-Rezeptor Liganden werden von der SmithKline Beecham Cooperation in der Anmeldung US2004102508 offenbart. Die beanspruchten Verbindungen finden ihre Verwendung für die Behandlung oder Prophylaxe von Schmerz, allergischen Reaktionen und neurodegenerativer Erkrankungen.

EP₄-Antagonisten (γ-Lactame) werden in der Anmeldung W003/103604 beansprucht (Applied Research Systems). Die Verbindungen binden ca. 60-fach besser an den EP₄- als an den EP₂-Rezeptor und werden unter anderem zur Behandlung von vorzeitigen Wehen, Dysmenorrhoe, Asthma, Unfruchtbarkeit oder Fertilitätsstörungen beansprucht. Von derselben Firma werden in den Anmeldungen W003/053923 (substituierte Pyrrolidine) oder W003/035064 (substituierte Pyrazolidione) Verbindungen für die Behandlung von Erkrankungen, die mit Prostaglandinen assoziiert sind, wie beispielsweise Infertilität, Hypertension und Osteoporose, beansprucht. Die Verbindungen binden an den EP₄-und an den EP₂ Rezeptor-Subtypen. In der Anmeldung W003/037433 werden ω-Cycloalkyl, 17 Heteroaryl-Prostaglandinderivate als EP₂-Rezeptor Antagonisten, insbesondere für die Behandlung von erhöhtem Augeninnendruck, beansprucht.

In der Anmeldung W003/064391 (Pfizer Products) werden Metabolite von [3-[[N-(4-tert-butylbenzyl)(pyridin-3-ylsulfonyl)amino]methyl] Essigsäure beschrieben, die die Bindung von [³H] Prostaglandin-E₂ an den EP₂-Rezeptor inhibieren. Die Verwendung dieser Metabolite zur Behandlung von Osteoporose wird offenbart.

Tani *et al.* beanspruchen in der Anmeldung US2005124577 8-Azaprostaglandinderivate für die Behandlung von immunologischen Erkrankungen, allergischen Erkrankungen, vorzeitigen Wehen, Abort usw. Die Verbindungen binden an den EP₂ und an den EP₄ Rezeptor.

In der europäischen Patentanmeldung EP 1306087 werden EP₂-Rezeptor Agonisten beschrieben, die ihre Verwendung bei der Behandlung der erektilen Dysfunktion finden (Ono Pharmaceuticals). Die gleiche Strukturklasse wird in dem europäischen Patent EP 860430 (Ono Pharmaceuticals) beschrieben, ihre Verwendung für die Herstellung eines Medikaments für die Behandlung von immunologischen Erkrankungen, Asthma und Abort wird beansprucht. In der W004/009117 werden EP₂- und EP₄-Rezeptor Agonisten für die Behandlung von Erkrankungen beschrieben, die verursacht werden durch die Uteruskontraktion, beispielsweise Menstruationsbeschwerden (Ono Pharmaceuticals).

In den Anmeldungen W003/74483 und W003/09872 werden Agonisten beschrieben, die gleichermaßen an den EP₂ und an den EP₄ Rezeptor binden (Ono Pharmaceuticals).

Die Agonisten des EP₂ und des EP₄ Rezeptors werden häufig im Zusammenhang mit der Behandlung der Osteoporose beschrieben (W099/19300 (Pfizer), US2003/0166631 (Dumont Francis), W003/77910 (Pfizer), WO03/4537 (Pfizer), WO03/74483 und WO03/09872 (Ono Pharmaceuticals)) und für die Glaukombehandlung (WO04/37813, WO04/37786, WO04/19938, WO03/103772, WO03/103664, WO03/40123, WO03/47513, WO03/47417 (Merck Frosst Canada)) und US6410591 und US6747037 (Allergan).

In der Patentanmeldung W004/12656 (Applied Research Systems) werden EP₂- Rezeptor Agonisten im Zusammenhang mit Inflammation beansprucht. In der Patentanmeldung W003/77919 (Merck & Co. Inc.) werden EP₄-Rezeptor Agonisten für die Behandlung der Fertilität beansprucht.

Aus verschiedenen Anmeldungen aus dem Stand der Technik sind Indolderivate (WO 95/27712, CEMAF; EP 1189583, Neurim Pharmaceuticals) oder aus dem europäischen Patent EP 1226138 (Affinium Pharmaceuticals) bekannt. Diese Verbindungen sind Fab I Inhibitoren mit antibakterieller Wirkung, bzw. finden als Hautschutzmittel oder Kosmetika Verwendung, sie haben allerdings keine Wirkung am EP₂ Rezeptor.

Bisher sind jedoch keine selektiven EP₂-Rezeptor Agonisten und Antagonisten bekannt, die die Prozesse regulieren, die letztendlich für die Ovulation, Fertilisierung, Nidation und Dezidualisierung verantwortlich sind und somit zur Förderung oder Hemmung der Fertilität beitragen.

Aufgabe der vorliegenden Erfindung ist es daher, stabile EP₂-Rezeptor Antagonisten zur Verfügung zu stellen.

Diese Aufgabe wird durch die Verwendung der Verbindungen der allgemeinen Formel I gelöst, 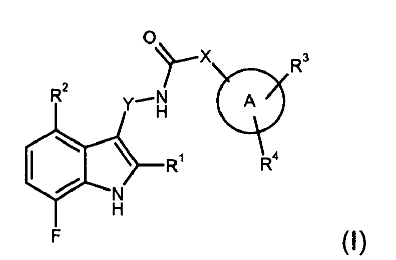 wobei,
- X: eine -CH=CH- oder -CH₂-CH₂-Gruppe und
- Y: eine -CH₂- Gruppe, bedeuten kann, wenn
- A: ein Phenyl- oder C₅-C₁₆-Heteroarylrest ist, der gegebenenfalls ein-oder mehrfach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, Amino, CF₃, Hydroxy, Cyano, ein C₁-C₄-Alkyl, C₁-C₄-Acyl, C₁-C₄-Alkoxy, CO₂-(C₁-C₄-Alkyl), N-(C₁-C₄-Alkyl)₂, CO-NH(C₁-C₄-Alkyl), CO-N(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
- R⁴: ein Wasserstoff, Halogen, Amino, CF₃, Hydroxy, Cyano, ein C₁-C₄-Alkyl, C₁-C₄-Acyl, ein C₁-C₄-Alkoxy, CO₂-(C₁-C₄-Alkyl), N-(C₁-C₄-Alkyl)₂, CO-NH(C₁-C₄-Alkyl), CO-N(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
oder wobei
- X: eine -CH=CH-Gruppe, und
- Y: eine -(CH₂)ₙ-Gruppe, wobei n für 2 oder 3 steht, bedeuten kann, wenn
- A: ein Phenylrest ist, der gegebenenfalls ein- oder zweifach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert ist mit Halogen, C₁-C₄-Alkyl oder C₁-C₄-Alkoxy,
- R⁴: ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert ist mit Halogen, C₁-C₄-Alkyl oder C₁-C₄-Alkoxy,
oder wobei
- X: eine-CH₂-CH₂- Gruppe, und
- Y: eine -(CH₂)ₙ-Gruppe, wobei n für 2 oder 3 steht, bedeuten kann wenn
- A: ein Phenyl- oder C₅-C₁₆-Heteroarylrest ist, der gegebenenfalls ein-oder mehrfach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung von Arzneimitteln für die Fertilitätskontrolle, die die bekannten Nachteile überwinden und verbesserte Eigenschaften, wie eine gute Wirksamkeit, gute Löslichkeit und Stabilität, aufweisen.

Die erfindungsgemäßen Verbindungen haben eine antagonistische Wirkung am EP₂-Rezeptor und dienen somit der weiblichen Fertilitätskontrolle.

Unter C₁-C₄-Alkyl ist jeweils ein geradkettiger oder verzweigter Alkylrest, wie beispielsweise Methyl, Ethyl, Propyl, Isopropyl, Butyl, Isobutyl, sek. Butyl, tert. Butyl, zu verstehen.

Unter C₁-C₄-Alkoxy ist jeweils ein geradkettiger oder verzweigter Alkoxyrest, wie beispielsweise Methoxy-, Ethoxy-, n-Propoxy-, iso-Propoxy-, n-Butoxy-, sec-Butoxy-, iso-Butoxy-, tert. Butyloxy-, zu verstehen.

Unter C₁-C₄-Acyl ist jeweils ein geradkettiger oder verzweigter Rest, wie beispielsweise Formyl, Acetyl, Propionyl, Butyroyl, iso-Butyroyl, zu verstehen. Unter Halogen ist jeweils Fluor, Chlor, Brom oder Jod zu verstehen.

Der C₅-C₁₆-Heteroarylrest umfasst jeweils 5 - 16 Ringatome und kann anstelle des Kohlenstoffs ein- oder mehrere, gleiche oder verschiedene Heteroatome, wie Sauerstoff, Schwefel oder Stickstoff im Ring enthalten, und kann mono-, bi-oder tricyclisch sein und kann zusätzlich jeweils benzokondensiert sein.

Beispielsweise seien genannt:
Thienyl, Furanyl, Pyrrolyl, Oxazolyl, Imidazolyl, Pyrazolyl, Isoxazolyl, Isothiazolyl, Oxadiazolyl, Triazolyl, Thiadiazolyl, etc. und Benzoderivate davon, wie z.B. Benzofuranyl, Benzothienyl, Benzooxazolyl, Benzimdazolyl, Indazolyl, Indolyl, Isoindolyl, etc; oder Pyridazinyl, Pyrimidinyl, Pyrazinyl, Triazinyl, etc. und Benzoderivate davon wie z.B. Chinolyl, Isochinolyl, etc; oder Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl, etc.

Der Heteroarylrest kann jeweils benzokondensiert sein. Beispielsweise seien als 5-Ringheteroaromaten genannt: Thiophen, Furan, Oxazol, Thiazol, Imidazol, Pyrazol und Benzoderivate davon und als 6-Ring-Heteroaromaten Pyridin, Pyrimidin, Triazin, Chinolin, Isochinolin und Benzoderivate.

Unter Heteroatomen sind Sauerstoff-, Stickstoff- oder Schwefel-Atome zu verstehen.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure u.a.

Ebenfalls Gegenstand der vorliegenden Erfindung ist die Verwendung von Verbindungen der allgemeinen Formel (I),
wobei
- X: eine -CH=CH- Gruppe, und
- Y: eine -(CH₂)ₙ-Gruppe, wobei n für 2 oder 3 steht, bedeuten kann, wenn
- A: ein Phenylrest ist, der gegebenenfalls ein- oder zweifach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
- R⁴: ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
oder wobei
- X: eine -CH₂-CH₂-Gruppe, und
- Y: eine -(CH₂)ₙ-Gruppe, wobei n für 2 oder 3 steht, bedeuten kann, wenn
- A: ein Phenyl- oder Heteroarylrest ist, der gegebenenfalls ein- oder mehrfach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

Ebenfalls Gegenstand der vorliegenden Erfindung ist die Verwendung von Verbindungen der allgemeinen Formel (I),
wobei
- X: eine -CH=CH- Gruppe, und
- Y: eine -(CH₂)₂- Gruppe bedeuten kann, wenn
- A: ein Phenylrest ist, der gegebenenfalls ein- oder zweifach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
- R⁴: ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy sein,
oder wobei
- X: eine -CH₂-CH₂-Gruppe, und
- Y: eine -(CH₂)₂- Gruppe bedeuten kann, wenn
- A: ein Phenyl- oder C₅-C₁₆-Heteroarylrest ist, der gegebenenfalls ein-oder mehrfach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

Ebenfalls Gegenstand der vorliegenden Erfindung ist die Verwendung von Verbindungen der allgemeinen Formel (I),
wobei
- X: eine -CH=CH- Gruppe, und
- Y: eine -(CH₂)₂- Gruppe bedeuten kann, wenn
- A: ein Phenylrest ist, der gegebenenfalls ein- oder zweifach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
- R⁴: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
- X: eine -CH₂-CH₂- Gruppe, und
- Y: eine -(CH₂)₂- Gruppe bedeuten kann, wenn
- A: ein Phenyl- oder C₅-C₁₆-Heteroarylrest ist, der gegebenenfalls ein-oder mehrfach mit R⁴ und/oder R³ substituiert ist,
- R¹: ein Wasserstoff oder ein C₁-C₄-Alkylrest ist, der gegebenenfalls substituiert ist,
- R²: ein Wasserstoff, Halogen, Cyano, ein C₁-C₄-Alkoxyrest oder C₁-C₄-Alkylrest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

Ebenfalls Gegenstand der vorliegenden Erfindung ist die Verwendung der Verbindungen der allgemeinen Formel (I),
wobei
- X: eine -CH=CH- Gruppe, und
- Y: eine -(CH₂)₂- Gruppe bedeuten kann, wenn
- A: ein Phenylrest ist, der gegebenenfalls ein- oder zweifach mit R⁴ und/oder R³ substituiert ist,
- R¹: ein Wasserstoff oder ein CH₃-Rest ist,
- R²: ein Wasserstoff, Fluor oder C₁-C₄-Alkylrest ist,
- R³: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
- R⁴: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
- X: eine-CH₂-CH₂- Gruppe, und
- Y: eine -(CH₂)₂- Gruppe, bedeuten kann, wenn
- A: ein Phenyl- oder Heteroarylrest ist, der gegebenenfalls ein- oder mehrfach mit R⁴ und/oder R³ substituiert ist,
- R¹: ein Wasserstoff oder ein CH₃-Rest ist,
- R²: ein Wasserstoff, Fluor oder C₁-C₄-Alkylrest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

Ebenfalls Gegenstand der vorliegenden Erfindung ist die Verwendung der Verbindungen der allgemeinen Formel (I),
wobei
- X: eine -CH=CH-Gruppe, und
- Y: eine -(CH₂)₂- Gruppe bedeuten kann, wenn
- A: ein Phenylrest ist, der gegebenenfalls ein- oder zweifach mit R⁴ und/oder R³ substituiert ist,
- R¹: ein Wasserstoff oder ein CH₃-Rest ist,
- R²: ein Wasserstoff, Fluor oder CH₃-Rest ist,
- R³: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
- R⁴: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
- X: eine-CH₂-CH₂- Gruppe, und
- Y: eine -(CH₂)₂- Gruppe bedeuten kann, wenn
- A: ein Phenyl- oder Heteroarylrest ist, der gegebenenfalls ein- oder mehrfach mit R⁴ und/oder R³ substituiert ist,
- R¹: ein Wasserstoff oder ein CH₃-Rest ist,
- R²: ein Wasserstoff, Fluor oder CH₃-Rest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

Ebenfalls Gegenstand der vorliegenden Erfindung ist die Verwendung der Verbindungen der allgemeinen Formel (I),
wobei,
- X: eine -CH=CH- Gruppe, und
- Y: eine -(CH₂)₂- Gruppe bedeuten kann, wenn
- A: ein Phenylrest ist, der gegebenenfalls ein- oder zweifach mit R⁴ und/oder R³ substituiert ist,
- R¹: ein Wasserstoff oder ein CH₃-Rest ist,
- R²: ein Wasserstoff, Fluor oder CH₃-Rest ist,
- R³: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
- R⁴: ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
- X: eine-CH₂-CH₂- Gruppe, und
- Y: eine -(CH₂)₂- Gruppe bedeuten kann, wenn
- A: ein Phenyl- oder Pyridylrest ist, der gegebenenfalls ein- oder mehrfach mit R⁴ und/oder R³ substituiert ist,
- R¹: ein Wasserstoff oder ein CH₃-Rest ist,
- R²: ein Wasserstoff, Fluor oder CH₃-Rest ist,
- R³: ein Wasserstoff, Halogen, C₁-C₄-Alkyl oder C₁-C₄-Alkoxy ist,
- R⁴: ein Wasserstoff, Halogen, C₁-C₄-Alkyl oder C₁-C₄-Alkoxy ist,
bedeuten, sowie deren Isomere, Diastereomere, Enantiomere und Salze bzw. Cyclodextrinclathrate, für die Herstellung eines Arzneimittels zur Fertilitätskontrolle.

Die folgenden Verbindungen entsprechend der vorliegenden Erfindung sind ganz besonders bevorzugt:
1. (E)-N-[2-(7-Fluoro-2,4-dimethyl-1 H-indol-3-yl)-ethyl]-3-(4-methoxy-phenyl)-acrylamid
2. (E)-3-(4-Chloro-phenyl)-N-[2-(7-fluoro-2,4-dimethyl-1 H-indol-3-yl)-ethyl]-acrylamid
3. (E)-N-[2-(7-Fluoro-2,4-dimethyl-1 H-indol-3-yl)-ethyl]-3-(3-methoxy-phenyl)-acrylamid
4. 3-(3-Ethoxy-phenyl)-N-[2-(7-fluoro-2,4-dimethyl-1H-indol-3-yl)-ethyl]-propionamid
5. N-[2-(7-Fluoro-2,4-dimethyl-1H-indol-3-yl)-ethyl]-3-pyridin-3-yl-propionamid
6. N-[2-(7-Fluoro-2,4-dimethyl-1 H-indol-3-yl)-ethyl]-3-(4-fluoro-phenyl)-propionamid
7. (E)-N-[2-(7-Fluor-2,4-dimethyl-1 H-indol-3-yl)-ethyl]-3-p-tolyl-acrylamid
8. (E)-3-(4-Brom-phenyl)-N-[2-(7-fluor-2,4-dimethyl-1H-indol-3-yl)-ethyl]-acrylamid
9. (E)-N-[2-(4,7-Difluor-2-methyl-1 H-indol-3-yl)-ethyl]-3-(4-methoxy-phenyl)-acrylamid
10. (E)-3-(3,4-Dimethoxy-phenyl)-N-[2-(7-fluor-2,4-dimethyl-1 H-indol-3-yl)-ethyl]-acrylamid
11. (E)-N-[2-(7-Fluor-2,4-dimethyl-1 H-indol-3-yl)-ethyl]-3-(2-methoxy-phenyl)-acrylamid
12. (E)-N-[2-(7-Fluor-2,4-dimethyl-1 H-indol-3-yl)-ethyl]-3-phenyl-acrylamid
13. (E)-N-[2-(7-Fluor-2,4-dimethyl-1 H-indol-3-yl)-ethyl]-3-m-tolyl-acrylamid
14. (E)-N-[2-(7-Fluor-2,4-dimethyl-1 H-indol-3-yl)-ethyl]-3-o-tolyl-acrylamid
15. (E)-N-[2-(7-Fluor-2,4-dimethyl-1 H-indol-3-yl)-ethyl]-3-(4-hydroxy-phenyl)-acrylamid
16. (E)-N-[2-(7-Fluor-2,4-dimethyl-1 H-indol-3-yl)-ethyl]-3-(3-fluoro-4-methoxyphenyl)-acrylamid
17. (E)-3-(4-Dimethylamino-phenyl)-N-[2-(7-fluor-2,4-dimethyl-1H-indol-3-yl)-ethyl]-acrylamid
18. (E)-3-(2,4-Difluor-phenyl)-N-[2-(7-fluor-2,4-dimethyl-1 H-indol-3-yl)-ethyl]-acrylamid
19. (E)-3-(3,5-Dimethyl-phenyl)-N-[2-(7-fluor-2,4-dimethyl-1 H-indol-3-yl)-ethyl]-acrylamid
20. (E)-3-(3,5-Difluor-phenyl)-N-[2-(7-fluor-2,4-dimethyl-1H-indol-3-yl)-ethyl]-acrylamid
21. (E)-3-(2-Chlor-phenyl)-N-[2-(7-fluor-2,4-dimethyl-1 H-indol-3-yl)-ethyl]-acrylamid
22. (E)-N-[2-(7-Fluor-2,4-dimethyl-1 H-indol-3-yl)-ethyl]-3-(3-hydroxy-phenyl)-acrylamid
23. (E)-3-(3,4-Difluor-phenyl)-N-[2-(7-fluor-2,4-dimethyl-1H-indol-3-yl)-ethyl]-acrylamid
24. (E)-N-[2-(7-Fluor-2,4-dimethyl-1 H-indol-3-yl)-ethyl]-3-(2-fluoro-phenyl)-acrylamid

Gegenstand der vorliegenden Erfindung ist die Verwendung der erfindungsgemäßen Verbindungen für die Herstellung von Arzneimitteln, die mindestens eine der Verbindungen gemäß Formel I enthalten.

Ebenfalls Gegenstand der vorliegenden Erfindung sind Arzneimittel, die die erfindungsgemäßen Verbindungen enthalten mit geeigneten Formulierungsund Trägerstoffen.

Im Vergleich zu bekannten Prostaglandin E₂-Liganden zeichnen sich die neuen EP₂-Agonisten und Antagonisten durch größere Selektivität und Stabilität aus.

Gegenstand der vorliegenden Erfindung sind Arzneimittel zur Behandlung und Prophylaxe von Erkrankungen, zu denen Fertilitätsstörungen, infektiöse Erkrankungen, Krebs, virale Infektionen, Herz-Kreislauf-Erkrankungen, erhöhter Augeninnendruck, Glaukoma, Erkrankungen des Knochenapparates, angiogenetische Erkrankungen, Störungen der Uteruskontraktion, Schmerz, neuroinflammatorische Erkrankungen, immunomodulatorische Infektionen und nephrologische Erkrankungen zählen.

Unter Fertilitätsstörungen sind die Erkrankungen zu verstehen, die dazu führen, dass keine Ovulation erfolgt, dass es nicht zur Nidation einer befruchteten Eizelle kommt und keine Dezidualisierung stattfindet, unter infektiösen Erkrankungen sind durch unizelluläre Parasiten hervorgerufene Erkrankungen, unter Krebs solide Tumoren und Leukämie, unter viralen Infektionen z. B. Cytomegalus-Infektionen, Hepatitis, Hepatitis B und C und HIV Erkrankungen, unter immunmodulatorischen Infektionen z.B. Vogelgrippe, unter Herz-Kreislauf-Erkrankungen ischämische Reperfusionserkrankung, Stenosen, Arteriosklerosen und Restenosen, unter angiogenetischen Erkrankungen z.B. Endometriose und Fibrose, unter erhöhtem Augeninnendruck Glaukom, unter Störungen der Uteruskontraktion z.B. Menstruationsbeschwerden, unter Erkrankungen des Knochenapparates Osteoporose, unter neuroinflammatorischen Erkrankungen Multiple Sklerose, Morbus Alzheimer, Schmerz und unter nephrologischen Erkrankungen Glomerulonephritis, zu verstehen.

Ebenfalls Gegenstand der vorliegenden Erfindung sind Arzneimittel zur Behandlung und Prophylaxe der oben aufgeführten Erkrankungen, die mindestens eine Verbindung gemäß der allgemeinen Formel I enthalten, sowie Arzneimittel mit geeigneten Formulierungs- und Trägerstoffen.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln, in halbfester Form, zum Beispiel als Salben, Cremes, Gele, Suppositiorien, Emulsionen oder in flüssiger Form, zum Beispiel als Lösungen, Suspensionen oder Emulsionen vorliegen.

Gegebenenfalls enthalten sie Hilfsstoffe, die beispielsweise als Füllstoffe, Bindemeittel, Sprengmittel, Gleitmittel, Lösungsmittel, Lösungsvermittler, Geschmackskorrigentien, Farbstoff, Emulgatoren, fungieren sollen. Hilfsstoffarten im Sinne der Erfindung sind beispielsweise Saccharide (Mono-, Di-, Tri-, Oligo-, und/oder Polysaccharide), Fette, Wachse, Öle, Kohlenwasserstoffe, anionische, nichtionische, kationische natürliche, synthetische oder halbsynthestische Tenside. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Zur Inhalation werden zweckmäßigerweise Ärosollösungen hergestellt.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist. Für die orale Anwendung derartiger Verbindungen sind ebenfalls auch Clathrate geeignet, beispielsweise seien genannt die Clathrate mit alpha-, beta-, gamma-Cyclodextrin oder auch beta-hydroxypropyl-Cyclodextrin.

Für die parenterale Verabreichung werden sterile, injizierbare, wässrige oder ölige Lösungen benutzt. Besonders geeignet sind Injektionslösungen oder Suspensionen, insbesondere wässrige Lösungen der aktiven Verbindungen in polyethoxyliertem Rizinusöl.

Für die vaginale Applikation sind z. B. Zäpfchen, Tampons oder intratauriner Device geeignet und üblich.

Für die intraartikuläre Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Ärosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01 % - 20 % betragen, um eine ausreichende pharmakologische Wirkung zu erzielen.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Die oben beschrieben Formulierungen und Darreichungsformen sind ebenfalls Gegenstand der vorliegenden Erfindung.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Die Administration der erfindungsgemäßen Verbindungen kann durch jede konventionelle Methode erfolgen, einschließlich oraler und parenteraler, z.B. durch subcutane oder intramuskuläre Injektionen. Die enteralen, parenteralen, vaginalen und oralen Applikationen sind ebenfalls Gegenstand der vorliegenden Erfindung.

Die erfindungsgemäßen Verbindungen der allgemeinen Formel I binden an den EP₂ Rezeptor und haben agonistische oder antagonistische Wirkung. Es kann durch einen Agonismustest (siehe Beispiel 1.2.1. der biologischen Beispiele) oder durch einen Antagonismustest (siehe Beispiel 1.2.2. der biologischen Beispiele) bestimmt werden, ob eine agonistische oder antagonistische Wirkung vorliegt.

Unter Antagonisten sind solche Moleküle zu verstehen, die an ihre entsprechenden Rezeptoren binden und welche die Initiierung des/der mit dem Rezeptor gekoppelten Signaltransduktionswege/s durch den oder die natürlich vorkommenden Liganden inhibieren. Üblicherweise kompetitieren die Antagonisten mit dem natürlich vorkommenden Liganden des Rezeptors um die Bindung an den Rezeptor. Aber auch andere Modifikationen des Rezeptors durch Moleküle, die verhindern, dass die mit dem Rezeptor gekoppelten Signaltransduktionswege durch den oder die natürlich vorkommenden Liganden aktiviert werden, sind möglich (z.B. nicht-kompetitive, sterische Modifikationen des Rezeptors).

Bevorzugterweise binden die Antagonisten reversibel an ihre korrespondierenden Rezeptoren.

Der EP₂ Rezeptor Antagonist hat eine bevorzugte Affinität für den EP₂ Rezeptor gegenüber jedem anderen EP-Rezeptor. Der Antagonismus wird in Gegenwart des natürlichen Agonisten gemessen (PGE₂).

Unter Agonisten sind solche Moleküle zu verstehen, die an ihre entsprechenden Rezeptoren binden und üblicherweise mit dem natürlich vorkommenden Liganden des Rezeptors um die Bindung an den Rezeptor kompetitieren und welche die Initiierung des mit dem Rezeptor gekoppelten Signaltransduktionsweges stimulieren. Agonisten können auch die Bindung des natürlichen Liganden unterstützen.
Rezeptoragonisten binden typischerweise selektiv an ihren bestimmten Rezeptor und nicht an andere Rezeptoren. Sie haben normalerweise eine höhere Bindungsaffinität als der natürliche Ligand. Obwohl Agonisten, die eine höhere Affinität zum Rezeptor haben als der natürliche Ligand, bevorzugt sind, können ebenfalls Agonisten mit einer geringeren Affinität eingesetzt werden.
Bevorzugterweise binden die Agonisten reversibel an ihre korrespondierenden Rezeptoren.

Der EP₂ Rezeptor Agonist hat eine bevorzugte Affinität für den EP₂ Rezeptor gegenüber jedem anderen EP-Rezeptor.

Agonisten werden über die Initiierung der entsprechenden Rezeptor vermittelten Signaltransduktion und/oder physiologischen Wirkung getestet.
Als Liganden werden die Verbindungen oder niedermolekulare Substanzen bezeichnet, die an einen Rezeptor binden. Ihre Bindung ist üblicherweise reversibel. Durch die Bindung eines Liganden an den entsprechenden Rezeptor wird der mit dem Rezeptor gekoppelte Signaltransduktionsweg aktiviert oder inaktiviert. Auf diese Art und Weise vermittelt der Ligand seine intrazelluläre Wirkung. Unter Liganden sind Agonisten und Antagonisten eines Rezeptors zu verstehen.

Die Substanz gemäß Beispiel 9 zeigt im zellulären Agonismustest keine Inhibition, im Antagonismustest jedoch eine gute Wirksamkeit (IC₅₀ = 1.5 x 10 E-6).
Ebenfalls Gegenstand der vorliegenden Erfindung ist die Verwendung der erfindungsgemäßen Substanzen als EP₂-Rezeptor Antagonisten für die Behandlung von Erkrankungen, die verursacht werden durch Störungen in der Signaltransduktionskette, an der der EP₂-Rezeptor beteiligt ist, wie beispielsweise Schmerz und Fertilitätsstörungen, und die ebenfalls geeignet sind für die Fertilitätskontrolle.

Im prä-ovulatorischen antralen Follikel ist die Eizelle von Kumulus Zellen umgeben, die einen dichten Zellkranz um die Eizelle bilden. Nach dem Peak des Lutenisierenden Hormons (LH-Peak) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.

Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al.. Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.
Die erfindungsgemäßen Substanzen haben inhibitorische Wirkungen in Kumulus Expansionstests.
Gegenstand der vorliegenden Erfindung ist die Verwendung der erfindungsgemäßen Substanzen zur Fertilitätskontrolle.

Während der EP₂-Rezeptor Antagonist AH 6809 die Expansion des Kumulus erst bei einer Konzentration von 100 - 200 µM um nur ca. 30 % unterdrückt, kann in Anwesenheit von der Substanz gemäß Beispiel 9 eine 20 %ige Unterdrückung der Kumulus Expansion bei einer 10 - 20-fach geringeren Konzentration (10 µM) erreicht werden. Bei diesen Versuchen kompetitieren die Testsubstanzen mit dem natürlichen EP₂-Rezeptor Agonisten PGE₂.
Gegenstand der vorliegenden Erfindung ist die Verwendung der erfindungsgemäßen Substanzen für die Hemmung der Kumulus Expansion und dadurch der Ovulation und Fertilisation zur Kontrazeption.

Prostaglandine spielen eine wichtige Rolle bei der Angiogenese (Sales, Jabbour, 2003, Reproduction 126, 559 - 567; Kuwano et al., 2004, FASEB J. 18, 300-310; Kamiyama et al., 2006, Oncogene 25, 7019-7028; Chang et al. 2005, Prostaglandins & other Lipid Mediators 76, 48-58).

Endometriose ist eine chronische Erkrankung, die verursacht wird durch Störungen der Blutgefäße. Circa 10 % der Frauen leiden regelmäßig an starken Blutungen während der Menstruation, verursacht durch Änderungen der Blutgefäße des Endometriums. Zusätzlich wurden strukturelle Unterschiede in den Blutgefäßen beobachtet, wie zum Beispiel die unvollständige Ausbildung der glatten Muskelzellschicht (Abberton et al., 1999, Hum. Reprod. 14, 1072-1079). Da der Blutverlust während der Menstruation zum Teil durch die Konstriktion der Blutgefäße geregelt wird, ist es naheliegend, dass die Defekte der glatten Muskulatur wesentlich zur Blutung beitragen.

Gegenstand der vorliegenden Erfindung ist die Verwendung der Substanzen der allgemeinen Formel I für die Behandlung von Endometriose.

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567).

Gegenstand der vorliegenden Erfindung ist die Verwendung der Substanzen der allgemeinen Formel I für die Behandlung von Menstruationsbeschwerden.

Zunehmende Forschungsergebnisse belegen auch die Bedeutung der EP-Rezeptoren, und insbesondere auch des EP₂-Rezeptoren bei einer Vielzahl von Krebsarten (z. B. Brustkrebs, Kolonkarzinom, Lungenkrebs, Prostatakrebs, Leukämie, Hautkrebs), was auf zukünftige Möglichkeiten des Einsatzes von Modulatoren (Antagonisten oder Agonisten) des EP2-Rezeptors für die Therapie und Vorbeugung (prophylaktisch und/oder adjuvant) von Krebs schließen lässt (Fulton et al. Cancer Res 2006; 66(20): 9794-7; Castellone et al. Science VOL 310 2005, 1504-1510; Chang et al. Cancer Res 2005; 65(11): 4496-9); Hull et al. Mol Cancer Ther 2004;3(8):1031-9; Richards et al. J Clin Endocrinol Metab 88: 2810-2816, 2003; Sinha et al. 2007, Cancer Res; 67(9):4507-13; Wang et al. 2004, Seminars in Oncology, Vol 31, No 1, Suppl 3: pp 64-73), Jain et al. Cancer Res 2006; 66(13): 6638-48).

Gegenstand der vorliegenden Erfindung ist die Verwendung der Substanzen der allgemeinen Formel I für die Behandlung und Vorbeugung von Krebserkrankungen.

Prostaglandine spielen auch eine wichtige Rolle bei den Prozessen, die dem Knochenschwund entgegen wirken. Gegenstand der vorliegenden Erfindung ist daher die Verwendung der erfindungsgemäßen Substanzen für die Behandlung von Knochenschwund.

Reinold et al. (J. Clin. Invest. 115, 673-679 (2005)) beschreibt PGE₂ Rezeptoren vom EP₂-Subtyp als die Schlüssel-Signalelemente bei der inflammatorischen Hyperalgesie. Mäuse, die diesen Rezeptor nicht mehr tragen (EP₂^{-/-}), empfinden keinen spinalen inflammatorischen Schmerz. Es gibt Hinweise, dass eine inflammatorische, gesteigerte Schmerzempfindlichkeit behandelt werden kann, indem gezielt EP₂-Rezeptoren moduliert werden.

Gegenstand der vorliegenden Erfindung ist die Verwendung der erfindungsgemäßen Substanzen für die Behandlung von inflammatorischer Hyperalgesie.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.

Die Herstellung der Salze erfolgt in üblicher Weise, indem man eine Lösung der Verbindung der Formel I mit der äquivalenten Menge oder einem Überschuss einer Base oder Säure, die gegebenenfalls in Lösung ist, versetzt und den Niederschlag abtrennt oder in üblicher Weise die Lösung aufarbeitet.

Die Erfindung betrifft damit auch Arzneimittel auf Basis der Verbindungen der allgemeinen Formel I und der üblichen Hilfs- oder Trägerstoffe.

Außerdem betrifft die Erfindung ein Verfahren zur Herstellung der erfindungsgemäßen Verbindungen der allgemeinen Formel I, das dadurch gekennzeichnet ist, dass man eine Verbindung der Formel II, 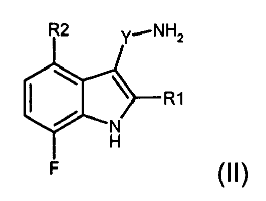 worin R¹, R² und Y die oben angegebenen Bedeutungen haben, mit einem Carbonsäurederivat der allgemeinen Formel III, 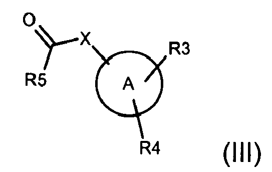 worin A, R³, R⁴ und X die oben angegebenen Bedeutungen aufweisen und R⁵ eine Hydroxygruppe, ein Chlor- oder Bromatom oder ein C₁-C₆-Akylrest sein kann, bevorzugt ist Wasserstoff, Chlor, der Methyl- oder Ethyl-Rest, nach dem Fachmann bekannten Methoden umsetzt und gegebenenfalls benötigte Schutzgruppen anschließend gespalten werden.

Für den Fall, dass R⁵ gleich eine Hydroxygruppe ist, kann die Umsetzung zunächst durch Aktivierung der Säurefunktion erfolgen, hierbei wird beispielsweise zunächst die Carbonsäure der Formel III in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amins erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen - 30 ° C und + 60 °C, vorzugsweise bei 0 ° C bis 30 ° C.

Eine weitere Möglichkeit besteht darin, die Carbonsäure durch Reagenzien wie beispielsweise HOBt oder HATU zu aktivieren. Die Umsetzung der Säure erfolgt z.B. mit HATU in einem inerten Lösungsmittel wie beispielsweise DMF in Gegenwart des entsprechenden Amins der allgemeinen Formel III und einem tertiären Amin wie beispielsweise Ethyldiisopropylamin bei Temperaturen zwischen - 50 und + 60 °C, vorzugsweise bei 0 ° C bis 30 ° C.

Für den Fall, dass R⁵ gleich C₁-C₆-Alkyl ist, kann beispielsweise auch eine direkte Amidolyse des Esters mit dem entsprechenden Amin eventuell unter zu Hilfenahme von Aluminiumtrialkyl-Reagenzien, vorzugsweise Aluminiumtrimethyl, durchgeführt werden.

Für den Fall, dass R⁵ gleich ein Chlor- oder Bromatom ist, kann beispielsweise die Umsetzung beispielsweise in Pyridin oder einem inerten Lösungsmittel wie beispielsweise DMF in Gegenwart des entsprechenden Amins der allgemeinen Formel II und einem tertiären Amin wie beispielsweise Ethyldiisopropylamin bei Temperaturen zwischen - 50 und + 60 ° C, vorzugsweise bei 0 °C bis 30 ° C, durchgeführt werden.

Die Verbindungen in denen X gleich -CH₂-CH₂- ist, können auch durch eine Hydrierung der entsprechenden Verbindung mit X = -CH=CH- durch die dem Fachmann bekannten Methoden wie beispielsweise Rühren in einer Wasserstoffatmosphäre in einem inerten Lösungsmittel, z. B. Tetrahydrofuran, Methylenchlorid, Methanol oder auch Essigester, in Gegenwart eines PalladiumKatalysators hergestellt werden.

Die als Ausgangsmaterialien dienenden Verbindungen der allgemeinen Formel II sind entweder bekannt oder können beispielsweise hergestellt werden, in dem man in an sich bekannter Weise die bekannten Hydrazine IV, gegebenenfalls hergestellt aus den entsprechenden bekannten Anilinen durch Nitrosierung gefolgt von einer Reduktion, 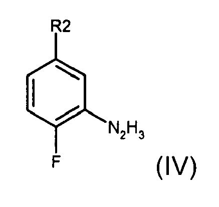 worin R² die oben angegebene Bedeutung aufweist,
a) mit einem Keton der allgemeinen Formel V, worin R¹ und Y die oben angegebene Bedeutung aufweist und n = 2 und 3 ist, in einer Fischer-Indol-Zyklisierung umsetzt, 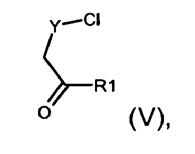 oder,
b) mit einem Enolether der allgemeinen Formel VI, worin R¹ und Y die oben angegebene Bedeutung aufweist und n = 2 und 3 ist, in einer Fischer-Indol-Zyklisierung umsetzt (Org. Lett. 2004, 79ff), 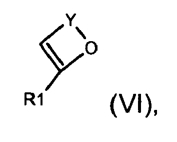 und der anschließend erhaltene Alkohol durch die dem Fachmann bekannten Methoden durch Überführung in eine Abgangsgruppe wie Tosylat, Mesylat, Trifluormesylat, Chlorid, Bromid oder Iodid und anschließende Umsetzung mit z. B. Natriumazid, gefolgt von einer Hydrolyse mittels PPh₃/H₂O in Tetrahydrofuran in die Aminofunktion überführt wird,
   oder
c) mit einem Ketoester der allgemeinen Formel VII, für den Fall von Y mit n = 1 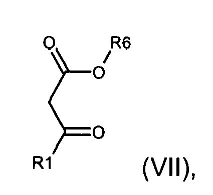 worin R¹ die oben angegebene Bedeutung hat und R⁶ ein C₁-C₆-Akylrest ist, in einer Fischer-Indol-Zyklisierung umgesetzt und anschließend der erhaltene Ester mit den dem Fachmann bekannten Methoden wie beispielsweise Diisobutylalumiumhydrid in einem inerten Lösungsmittel bei Temperaturen zwischen - 50 und 25 ° C, vorzugsweise zwischen - 30 und 0 ° C zum entsprechenden Alkohol reduziert wird, der wiederum durch Überführung in eine Abgangsgruppe wie Tosylat, Mesylat, Trifluormesylat, Chlorid, Bromid oder Iodid und anschließende Umsetzung mit z.B. Natriumazid, gefolgt von einer Hydrolyse mittels PPh₃/H₂O in Tetrahydrofuran in die Aminofunktion überführt wird.

Die Erfindung betrifft außerdem ein alternatives Verfahren zur Herstellung der erfindungsgemäßen Verbindungen der allgemeinen Formel I, das dadurch gekennzeichnet ist, dass man eine Verbindung der Formel II, 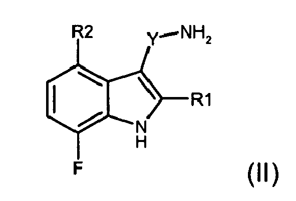 worin R¹, R² und Y die oben angegebenen Bedeutungen haben, zunächst mit Acrylsäurechlorid zu den Verbindungen der allgemeinen Formel VIII umsetzt

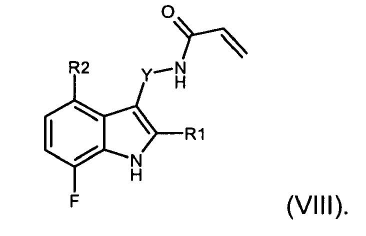

Die Verbindungen der allgemeinen Formel VIII werden dann in einer dem Fachmann bekannten Heck-Reaktion unter Verwendungen von Palladium-Katalysatoren wie beispielsweise Pd(OAc)₂, Pd₂(dba)₃, PdCl₂ oder PdCl₂(PhCN)₂ mit Verbindungen der allgemeinen Formel IX 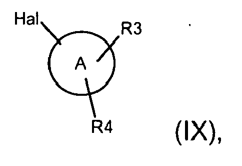 worin A, R³ und R⁴ die oben angegebenen Bedeutungen aufweisen und Hal für ein Chlor-, Brom- oder lodatom steht, bevorzugt ist ein Bromatom, zu den Verbindungen der allgemeinen Formel I umgesetzt und in der gegebenenfalls für den Fall Y = -CH₂-CH₂- die vorhandene Doppelbindung durch die dem Fachmann bekannten Methoden, wie beispielsweise Rühren in einer Wasserstoffatmosphäre in einem inerten Lösungsmittel z. B. Tetrahydrofuran, Methylenchlorid, Methanol oder auch Essigester in Gegenwart eines PalladiumKatalysators, hydriert werden muss und gegebenenfalls benötigte Schutzgruppen anschließend gespalten werden.

Gegebenenfalls können die Verbindungen der allgemeinen Formel (I) mit R² = CN auch ausgehend von den entsprechenden Halogeniden, bevorzugt ist Brom bzw. Chlor, durch eine Cu- oder Pd-katalysierte (z.B. Pd(AOc)₂) Cyanid-Einführung mit Zn(CN)₂ oder auch K₃[Fe(CN)₆] in einem inerten Lösungsmittel wie Dimethylacetamid, Dimethylforamid oder N-Methylpyrolidon bei Temperaturen zwischen 60 ° C und dem Siedepunkt des jeweiligen Lösungsmittels erfolgen.

### Herstellung der erfindungsgemäßen Verbindungen

Die nachfolgenden Beispiele erläutern die Herstellung der erfindungsgemäßen Verbindungen der allgemeinen Formel (I), ohne den Umfang der beanspruchten Verbindungen auf diese Beispiele zu beschränken.

Die erfindungsgemäßen Verbindungen der allgemeinen Formel (I) lassen sich wie nachstehend beschrieben herstellen.

### Beispiel 1: (E)-N-[2-(7-Fluor-2,4-dimethyl-1 H-indol-3-yl)-ethyl]-3-(4-methoxyphenyl)-acrylamid

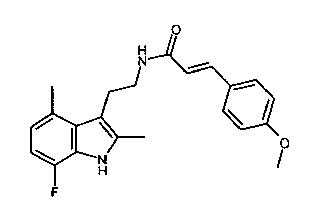

Zu einer Lösung von 294mg (E)-3-(4-Methoxy-phenyl)-acrylsäure in 12ml Dimethylformamid gibt man 689mg N-[(Dimethylamino)-1*H*-1,2,3-triazolo[4,5-*b*]pyridin-1-ylmethylen]-*N*-Methylmethanaminiumhexafluorophosphat-N-oxid (HATU) und 400 mg 2-(7-Fluoro-2,4-dimethyl-1 H-indol-3-yl)-ethylamin Hydrochlorid. Bei 0 ° C werden dann 0.6 ml Ethyldiisopropylamin zugetropft und 20 Stunden bei 25 ° C gerührt. Anschließend gibt man 50 ml eines Gemisches aus Eis und konz. wässriger Bicarbonat-Lösung zu und extrahiert dreimal mit Ethylacetat. Die vereinigten organischen Phasen wäscht man einmal mit gesättigter Natriumchlorid-Lösung, trocknet über Natriumsulfat und engt nach Filtration im Vakuum ein. Der so erhaltene Rückstand wird durch Chromatographie an Kieselgel mit Hexan / 0 - 100% Ethylacetat gereinigt. Man erhält auf diese Weise 211 mg der Titelverbindung.
NMR (300 MHz, DMSO-d6): δ = 2.28 (s, 3H), 2.56 (3H), 2.88 (2H), 3.24 (2H), 3.01 (3H), 6.43 (1 H), 6.56 (1 H), 6.62 (1 H), 6.94 (2H), 7.35 (1 H), 7.47 (2H), 8.15 (1H), 11.13 (1H).

### Beispiel 2: (E)-N-[2-(7-Fluor-2,4-dimethyl-1H-indol-3-yl)-ethyl]-3-p-tolyl-acrylamid

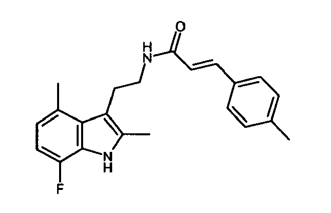

In Analogie zu Beispiel 1 erhält man aus 67 mg (E)-3-(4-Methyl-phenyl)-acrylsäure und 100 mg 2-(7-Fluor-2,4-dimethyl-1 H-indol-3-yl)-ethylamin Hydrochlorid 131 mg der Titelverbindung.
NMR (300 MHz, DMSO-d6): δ = 2.28 (6H), 2.56 (3H), 2.88 (2H), 3.24 (2H), 6.52 (1 H), 6.54-6.65 (2H), 7.19 (2H), 7.36 (1 H), 7.41 (2H), 8.20 (1 H), 11.13 (1 H).

### Beispiel 3: (E)-3-(4-Brom-phenyl)-N-[2-(7-fluor-2,4-dimethyl-1 H-indol-3-yl)-ethyl]-acrylamid

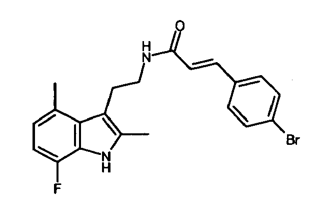

In Analogie zu Beispiel 1 erhält man aus 94 mg (E)-3-(4-Bromphenyl)-acrylsäure und 100 mg 2-(7-Fluor-2,4-dimethyl-1 H-indol-3-yl)-ethylamin Hydrochlorid 64 mg der Titelverbindung.
NMR (300 MHz, DMSO-d6): δ = 2.28 (3H), 2.56 (3H), 2.88 (2H), 3.24 (2H), 6.52-6.67 (3H), 7.38 (1 H), 7.48 (2H), 7.57 (2H), 8.26 (1H), 11.13 (1 H).

### Beispiel 4: (E)-N-[2-(4,7-Difluor-2-methyl-1 H-indol-3-yl)-ethyl]-3-(4-methoxyphenyl)-acrylamid

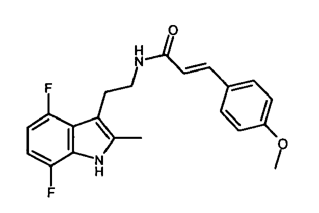

In Analogie zu Beispiel 1 erhält man aus 85mg (E)-3-(4-Methoxy-phenyl)-acrylsäure und 100mg 2-(4,7-Difluor-2-methyl-1 H-indol-3-yl)-ethylamin 43mg der Titelverbindung.
NMR (300 MHz, DMSO-d6): δ = 2.25 (3H), 2.82 (2H), 3.30 (2H), 3.74 (3H), 6.41 (1H), 6.60 (1H), 6.72 (1H), 6.93 (2H), 7.32 (1H), 7.45 (2H), 8.05 (1H), 11.46 (1 H).

Das Ausgangsmaterial für die obige Titelverbindung wird wie folgt hergestellt:
4a) 2-(4,7-Difluor-2-methyl-1 H-indol-3-yl)-ethylamin

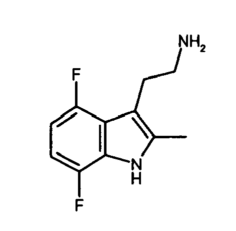

2.0 g 2,5-Difluorphenylhydrazin werden in 45 ml eines Gemisches aus Ethanol und Wasser im Verhältnis 14 : 1 bei 120 ° C gelöst. Anschließend gibt man in der Siedehitze 1.59 ml 5-Chlor-2-pentanon gelöst in 2 ml Ethanol hinzu und rührt bei dieser Temperatur für 16 Stunden. Nach dem Abkühlen wird im Vakuum eingeengt und der erhaltene Rückstand durch Säulenchromatographie an Kieselgel mit Methylenchlorid / 0 - 20 % methanol / 0.5 % Triethylamin gereinigt. Man erhält auf diese Weise 516 mg der Titelverbindung.
NMR (300 MHz, DMSO-d6): δ = 2.31 (3H), 2.85 (4H), 6.61 (1 H), 6.74 (1H), 11.56 (1 H).

### Allgemeine Vorschrift für die Heck-Reaktion

Zu einer Lösung von 255 mg N-[2-(7-Fluor-2,4-dimethyl-1 H-indol-3-yl)-ethyl]-acrylamid in 2 ml Dimethylformamid gibt man 49 mg Palladiumdiacetat, 232 mg Tri-ortho-tolylphosphin, 0.32 ml Triethylamin und 0.87 mmol des entsprechenden Bromids und rührt für 4 Stunden bei 110 ° C. Nach dem Abkühlen gibt man die Reaktionsmischung auf Wasser und extrahiert dreimal mit Ethylacetat. Die vereinigten organischen Phasen wäscht man einmal mit gesättigter Natriumchlorid-Lösung, trocknet über Natriumsulfat und engt nach Filtration im Vakuum ein. Der so erhaltene Rückstand wird durch HPLC (reversed phase) Chromatographie mit Wasser / 1 - 99 % Acetonitril plus 1 % TFA gereinigt. Man erhält auf diese Weise die entsprechenden Titelverbindungen.

Das Ausgangsmaterial für die obige Heck-Reaktion wird wie folgt hergestellt:
N-[2-(7-Fluor-2,4-dimethyl-1 H-indol-3-yl)-ethyl]-acrylamid

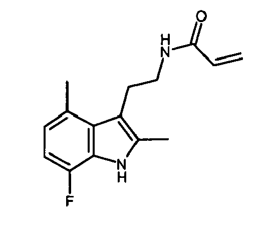

Zu einer Lösung von 8.0 g 2-(7-Fluor-2,4-dimethyl-1H-indol-3-yl)-ethylamin Hydrochlorid in 240 ml Dimethylformamid gibt man bei 25 °C 11.4 ml Triethylamin gefolgt von 2.67 ml Acrylsäurechlorid. Nach 60 min. Rühren bei 25 ° C gibt man 50 ml die Reaktionsmischung auf Eiswasser und extrahiert dreimal mit je 200 ml Ethylacetat. Die vereinigten organischen Phasen wäscht man zweimal mit gesättigter Wasser, trocknet über Natriumsulfat und engt nach Filtration im Vakuum ein. Der so erhaltene Rückstand wird durch Chromatographie an Kieselgel mit Hexan / 0 - 100 % Ethylacetat gereinigt. Man erhält auf diese Weise 5.84 g der Titelverbindung.
NMR (300 MHz, DMSO-d6): δ = 2.31 (3H), 2.58 (3H), 2.90 (2H), 3.24 (2H), 5.59 (1 H), 6.09 (1 H), 6.22 (1 H), 6.56-6.71 (2H), 8.27 (1 H), 11.17 (1 H).

In Analogie zu Beispiel 1 bzw. der allgemeinen Vorschrift der Heck-Reaktion werden die folgenden Beispiele hergestellt und per HPLC gereinigt:
HPLC-Methode:
HPLC-MS (analytisch) des gereinigten Produktes
Gerät: analytisches 4-Kanal MUX-System mit CTC Pal Injektor, Waters 1525 Pumpen, Waters 2488 UV-Detektor und Waters ZQ 2000 single quad MS Detektor
Säule X-Bridge RP C18 4.6x50 3.5µm; Detektionswellenlänge 214 nm; Flussrate 2 ml/min; Eluenten A: 0.1% TFA in H₂O, B 0.1% TFA in ACN;
Gradient jeweils bezogen auf B: 1 % auf 99% (5') auf 99% (1') auf 1 % (0.25') auf 1%(1.75')
HPLC Bedingungen für mit * gekennzeichnete Verbindungen:
Säule: Purospher STAR RP18e, 4x125mm, 5 µ (Merck KgGa, Darmstadt); Flussmittel: A: H₂O/0.1% TFA, B: CH₃CN/0.1% TFA, Gradient: 5 bis 95 % B in 10 min; Flussrate: 1 ml/min):

| Beispiel | Struktur | Name | Theoretische Masse m/z [M+H]⁺ | Gefundene Masse m/z [M+H]⁺ | Retentionszeit [min.] |
|---|---|---|---|---|---|
| 5 | 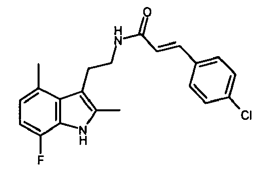 | (E)-3-(4-Chlorophenyl)-N-[2-(7-fluoro-2,4-dimethyl-1 H-indol-3-yl)-ethyl]-acrylamid | 370 | 370 | 9.92* |
| 6 | 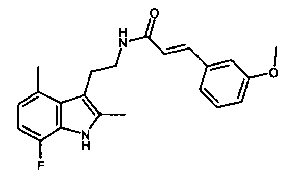 | (E)-N-[2-(7-Fluoro-2,4-dimethyl-1 H-indol-3-yl)-ethyl]-3-(3-methoxyphenyl)-acrylamid | 366 | 366 | 9.4* |
| 7 | 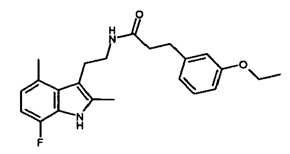 | 3-(3-Ethoxyphenyl)-N-[2-(7-fluoro-2,4-dimethyl-1 H-indol-3-yl)-ethyl]-propionamid | 382 | 382 | 9.52* |
| 8 | 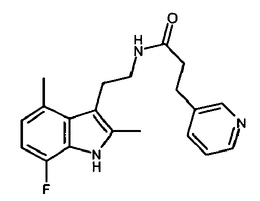 | N-[2-(7-Fluoro-2,4-dimethyl-1 H-indol-3-yl)-ethyl]-3-pyridin-3-yl-propionamid | 339 | 339 | 5.9* |
| 9 | 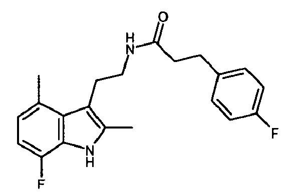 | N-[2-(7-Fluoro-2,4-dimethyl-1 H-indol-3-yl)-ethyl]-3-(4-fluorophenyl)-propionamid | 356 | 356 | 9.32* |
| 10 | 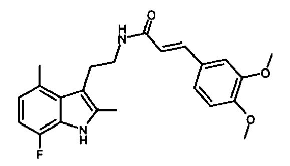 | (E)-3-(3,4-Dimethoxyphenyl)-N-[2-(7-fluor-2,4-dimethyl-1H-indol-3-yl)-ethyl]-acrylamid | 396,4595 | 397 | 9,21* |
| 11 | 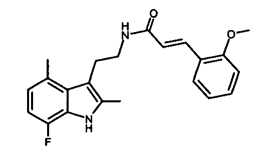 | (E)-N-[2-(7-Fluor-2,4-dimethyl-1 H-indol-3-yl)-ethyl]-3-(2-methoxyphenyl)-acrylamid | 366,4337 | 367 | 9,88* |
| 12 | 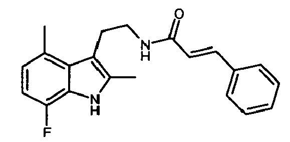 | (E)-N-[2-(7-Fluor-2,4-dimethyl-1 H-indol-3-yl)-ethyl]-3-phenylacrylamid | 336,4079 | 337 | 4,04 |
| 13 | 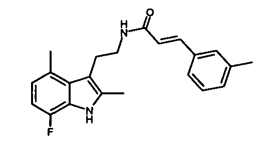 | (E)-N-[2-(7-Fluor-2,4-dimethyl-1 H-indol-3-yl)-ethyl]-3-m-tolylacrylamid | 350,4347 | 351 | 4,19 |
| 14 | 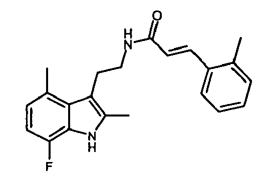 | (E)-N-[2-(7-Fluor-2,4-dimethyl-1 H-indol-3-yl)-ethyl]-3-o-tolylacrylamid | 350,4347 | 351 | 4,16 |
| 15 | 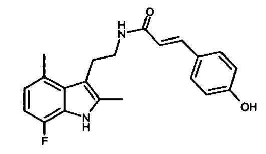 | (E)-N-[2-(7-Fluor-2,4-dimethyl-1 H-indol-3-yl)-ethyl]-3-(4-hydroxyphenyl)-acrylamid | 352,4069 | 353 | 3,59 |
| 16 | 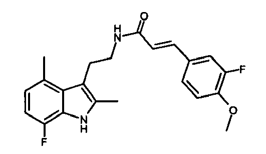 | (E)-N-[2-(7-Fluor-2,4-dimethyl-1H-indol-3-yl)-ethyl]-3-(3-fluoro-4-methoxy-phenyl)-acrylamid | 384,4238 | 385 | 4,02 |
| 17 | 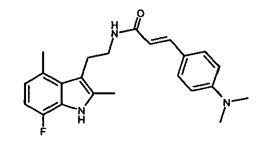 | (E)-3-(4-Dimethylam ino-phenyl)-N-[2-(7-fluor-2,4- dimethyl-1 H-indol-3-yl)-ethyl]-acrylamid | 379,4764 | 380 | 3,58 |
| 18 | 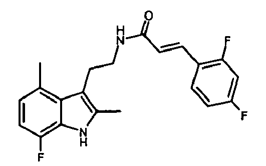 | (E)-3-(2,4-Difluophenyl)-N-[2-(7-fluor-2,4-dimethyl-1 H-indol-3-yl)-ethyl]-acrylamid | 372,3881 | 373 | 4,18 |
| 19 | 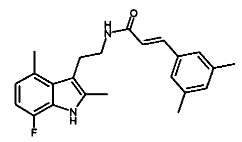 | (E)-3-(3,5-Dimethylphenyl)-N-[2-(7-fluor-2,4-dimethyl-1 H-indol-3-yl)-ethyl]-acrylamid | 364,4615 | 365 | 4,42 |
| 20 | 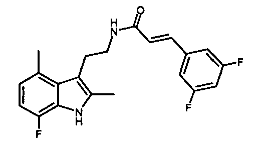 | (E)-3-(3,5-Difluorphenyl)-N-[2-(7-fluor-2,4-dimethyl-1 H-indol-3-yl)-ethyl]-acrylamid | 372,3881 | 373 | 4,24 |
| 21 | 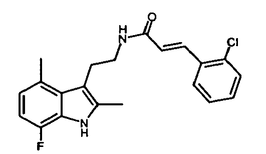 | (E)-3-(2-Chlor-phenyl)-N-[2-(7-fluor-2,4-dimethyl-1 H-indol-3-yl)-ethyl]-acrylamid | 370,853 | 372 | 4,28 |
| 22 | 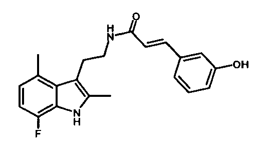 | (E)-N-[2-(7-Fluor-2,4-dimethyl-1 H-indol-3-yl)-ethyl]-3-(3-hydroxyphenyl)-acrylamid | 352,4069 | 353 | 3,61 |
| 23 | 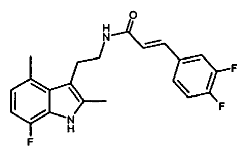 | (E)-3-(3,4-Difluorphenyl)-N-[2-(7-fluor-2,4-dimethyl-1 H-indol-3-yl)-ethyl]-acrylamid | 372,3881 | 373 | 4,14 |
| 24 | 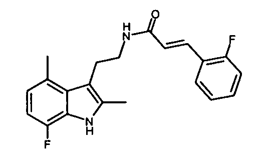 | (E)-N-[2-(7-Fluor-2,4-dimethyl-1 H-indol-3-yl)-ethyl]-3-(2-fluorophenyl)-acrylamid | 354,398 | 355 | 4,1 |

### Biologische Beispiele:

### 1. Nachweis des Antagonismus des humanen Prostaglandin E₂ (Subtyp EP₂) Rezeptorsignals

### 1.1 Nachweisprinzip

Die Bindung von PGE₂ an den EP₂-Subtyp des humanen PGE₂-Rezeptors induziert die Aktivierung membranständiger Adenylatzyklasen und führt zur Bildung von cAMP. In Gegenwart des Phosphodiesteraseinhibitors IBMX wird das durch diese Stimulation akkumulierte und mittels Zell-Lyse freigesetzte cAMP in einem kompetitiven Nachweisverfahren eingesetzt. In diesem Test konkurriert das cAMP im Lysat mit cAMP-XL665 um die Bindung eines Eu-Kryptat markierten Anti-cAMP Antikörpers.
Dabei entsteht in Abwesenheit von zellulärem cAMP ein maximales Signal, welches auf der Kopplung dieses Antikörpers an das cAMP-XL665 Molekül beruht. Dadurch entsteht nach Anregung bei 337 nm ein auf FRET (fluorescence resonance energy transfer) basierendes, lang anhaltendes Emissionssignal bei 665 nm (und bei 620 nM). Beide Signale werden in einem geeigneten Messgerät zeitlich versetzt, d.h. nach Abklingen der Hintergrundsfluoreszenz gemessen. Jegliche Erhöhung des durch Prostglandin-E₂-Gabe bedingten niedrigen FRET-Signals (gemessen als Well-Ratio-Veränderung = Emission₆₆₅ₙₘ/Emission₆₂₀ₙₘ * 10000) zeigt die Wirkung von Antagonisten.

### 1.2. Nachweisverfahren

### 1.2.1. Test auf Antagonismus (Angaben pro Vertiefung einer 384-Loch-Platte):

Die in einer Testplatte und 30 % DMSO vorgelegten Substanzlösungen (0.75 µl) werden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend werden 15 µl davon in eine medienfreie Zellkulturplatte überführt, die kurz zuvor mit KRSB gewaschen worden war.
Nach einer 30-minütigen Vorinkubation bei Raumtemperatur (RT) werden 5 µl einer 4xPGE₂ Lösung (11 nM) zugegeben und in Gegenwart des Agonisten für weitere 60 min bei RT inkubiert (Volumen: - 20 µl), bevor die Reaktion anschließend durch Zugabe von 5 µl Lysispuffer gestoppt und für weitere 20 min bei RT inkubiert wird (Volumen: - 25 µl). Das Zell-Lysat wird anschließend in eine Messplatte überführt und entsprechend den Herstellerangaben (cyclic AMP kit Cisbio International # 62AMPPEC) gemessen.

### 1.2.2. Test auf Agonismus (Angaben pro Vertiefung einer 384-Loch-Platte):

Die in einer in einer Testplatte und 30 % DMSO vorgelegten Substanzlösungen (0.75 µl) werden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend werden 15 µl davon in eine medienfreie Zellkulturplatte überführt, die kurz zuvor mit KRSB gewaschen worden war.
Nach einer 60-minütigen Inkubation bei Raumtemperatur (RT; Volumen: - 15 µl) wird die Reaktion anschließend durch Zugabe von 5 µl Lysisbuffer gestoppt und für weitere 20 min bei RT inkubiert (Volumen: - 20 µl). Das Zell-Lysat wird anschließend in eine Messplatte überführt und entsprechend den Herstellerangaben (cyclic AMP kit Cisbio International # 62AMPPEC) gemessen.

### 2. Der EP₂-Subtyp des PGE₂-Rezeptors und die prä-ovulatorische Kumulus Expansion

### 2.1. Hintergrund:

Im prä-ovulatorischen antralen Follikel ist die Eizelle von Kumulus Zellen umgeben, die einen dichten Zellkranz um die Eizelle bilden. Nach dem LH-Peak (Lutenisierendes Hormon) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.
Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al. Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.

### 2.2. Kumulus Expansions Test in vitro

In immaturen weiblichen Mäusen (Stamm : CD1 (ICR) von Charles River) wird in einem Alter von 14-18 Tagen die Follikulogenese durch eine einmalige Gabe (intraperitoneal) von 10 I.E. PMSG (Pregnant Mare Serum Gonadotropine; Sigma G-4877, Lot 68H0909) induziert. 47 - 50 Stunden nach der Injektion werden die Ovarien entnommen und die Kumulus-Eizell Komplexe entnommen. Der Kumulus Komplex ist in diesem Stadium noch nicht expandiert.
Die Kumulus-Eizell Komplexe werden nun für 20 - 24 Stunden mit Prostaglandin E₂ (PGE₂) (0,3 µM), Vehikel Kontrolle (Ethanol) oder Testsubstanzen inkubiert. Medium: alpha- MEM Medium mit 0,1 mM IBMX, Pyruvate (0,23 mM) Glutamine (2 mM), Pen/Strep 100 IU/ml Pen. und 100 µg/ml Strep.) und HSA (8 mg/ml)). Danach wird die Kumulus Expansion durch die Einteilung in vier Stadien (nach Vanderhyden et al. Dev Biol. 1990 Aug;140(2):307-317) festgestellt.

**Tabelle 1: Beispiel für die biologische Wirksamkeit der erfindungsgemäßen Verbindungen (gemessen mittels cAMP Antagonismustest):**

| Substanz gemäß Beispiel | Antagonismus [IC₅₀, µM] |
|---|---|
| 7 | 1.2 |
| 9 | 1.5 |
| 20 | 0.83 |

## Patentansprüche

1. Verbindungen der allgemeinen Formel I 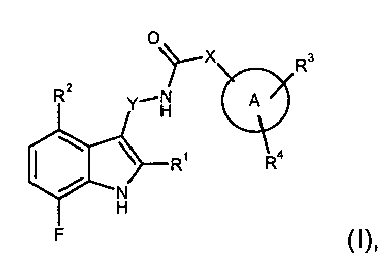 wobei
X eine -CH=CH- oder -CH₂-CH₂-Gruppe und
Y eine -CH₂- Gruppe, bedeuten kann, wenn
A ein Phenyl- oder C₅-C₁₆-Heteroarylrest ist, der gegebenenfalls ein-oder mehrfach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, Amino, CF₃, Hydroxy, Cyano, ein C₁-C₄-Alkyl, C₁-C₄-Acyl, C₁-C₄-Alkoxy, CO₂-(C₁-C₄-Alkyl), N-(C₁-C₄-Alkyl)₂, CO-NH(C₁-C₄-Alkyl), CO-N(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
R⁴ ein Wasserstoff, Halogen, Amino, CF₃, Hydroxy, Cyano, ein C₁-C₄-Alkyl, C₁-C₄-Acyl, ein C₁-C₄-Alkoxy, CO₂-(C₁-C₄-Alkyl), N-(C₁-C₄-Alkyl)₂, CO-NH(C₁-C₄-Alkyl), CO-N(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
oder wobei,
X eine -CH=CH-Gruppe, und
Y eine -(CH₂)ₙ-Gruppe, wobei n für 2 oder 3 steht, bedeuten kann, wenn
A ein Phenylrest ist, der gegebenenfalls ein- oder zweifach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert ist mit Halogen, C₁-C₄-Alkyl oder C₁-C₄-Alkoxy,
R⁴ ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert ist mit Halogen, C₁-C₄-Alkyl oder C₁-C₄-Alkoxy,
oder wobei,
X eine-CH₂-CH₂- Gruppe, und
Y eine -(CH₂)ₙ-Gruppe, wobei n für 2 oder 3 steht, bedeuten kann wenn
A ein Phenyl- oder C₅-C₁₆-Heteroarylrest ist, der gegebenenfalls ein-oder mehrfach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung von Arzneimittel für die Fertilitätskontrolle.

2. Verbindungen gemäß Anspruch 1,
wobei
X eine -CH=CH- Gruppe, und
Y eine -(CH₂)ₙ-Gruppe, wobei n für 2 oder 3 steht, bedeuten kann, wenn
A ein Phenylrest ist, der gegebenenfalls ein- oder zweifach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
R⁴ ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
oder wobei
X eine -CH₂-CH₂-Gruppe, und
Y eine -(CH₂)ₙ-Gruppe, wobei n für 2 oder 3 steht, bedeuten kann, wenn
A ein Phenyl- oder Heteroarylrest ist, der gegebenenfalls ein- oder mehrfach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

3. Verbindungen gemäß Anspruch 1 - 2,
wobei
X eine -CH=CH- Gruppe, und
Y eine -(CH₂)₂- Gruppe bedeuten kann, wenn
A ein Phenylrest ist, der gegebenenfalls ein- oder zweifach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy,
R⁴ ein Wasserstoff, Halogen, Amino, CF₃, Hydroxy, ein C₁-C₄-Alkyl, ein C₁-C₄-Alkoxy, N-(C₁-C₄-Alkyl)₂ oder ein Phenylrest ist, der gegebenenfalls ein- bis dreifach, gleich oder verschieden substituiert sein kann mit Halogen, C₁-C₄-Alkyl oder C₁-C₄-Alkoxy sein,
oder wobei
X eine -CH₂-CH₂-Gruppe, und
Y eine -(CH₂)₂- Gruppe bedeuten kann, wenn
A ein Phenyl- oder C₅-C₁₆-Heteroarylrest ist, der gegebenenfalls ein-oder mehrfach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

4. Verbindungen gemäß Anspruch 1 - 3,
wobei
X eine -CH=CH- Gruppe, und
Y eine -(CH₂)₂- Gruppe bedeuten kann, wenn
A ein Phenylrest ist, der gegebenenfalls ein- oder zweifach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
R⁴ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
X eine -CH₂-CH₂- Gruppe, und
Y eine -(CH₂)₂- Gruppe bedeuten kann, wenn
A ein Phenyl- oder C₅-C₁₆-Heteroarylrest ist, der gegebenenfalls ein-oder mehrfach mit R⁴ und/oder R³ substituiert ist,
R¹ ein Wasserstoff oder ein C₁-C₄-Alkylrest ist, der gegebenenfalls substituiert ist,
R² ein Wasserstoff, Halogen, Cyano, ein C₁-C₄-Alkoxyrest oder C₁-C₄-Alkylrest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

5. Verbindungen gemäß Anspruch 1 - 4,
wobei
X eine -CH=CH- Gruppe, und
Y eine -(CH₂)₂- Gruppe bedeuten kann, wenn
A ein Phenylrest ist, der gegebenenfalls ein- oder zweifach mit R⁴ und/oder R³ substituiert ist,
R¹ ein Wasserstoff oder ein CH₃-Rest ist,
R² ein Wasserstoff, Fluor oder C₁-C₄-Alkylrest ist,
R³ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
R⁴ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
X eine-CH₂-CH₂- Gruppe, und
Y eine -(CH₂)₂- Gruppe, bedeuten kann, wenn
A ein Phenyl- oder Heteroarylrest ist, der gegebenenfalls ein- oder mehrfach mit R⁴ und/oder R³ substituiert ist,
R¹ ein Wasserstoff oder ein CH₃-Rest ist,
R² ein Wasserstoff, Fluor oder C₁-C₄-Alkylrest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

6. Verbindungen gemäß Anspruch 1 - 5,
wobei
X eine -CH=CH-Gruppe, und
Y eine -(CH₂)₂- Gruppe bedeuten kann, wenn
A ein Phenylrest ist, der gegebenenfalls ein- oder zweifach mit R⁴ und/oder R³ substituiert ist,
R¹ ein Wasserstoff oder ein CH₃-Rest ist,
R² ein Wasserstoff, Fluor oder CH₃-Rest ist,
R³ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
R⁴ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
X eine-CH₂-CH₂- Gruppe, und
Y eine -(CH₂)₂- Gruppe bedeuten kann, wenn
A ein Phenyl- oder Heteroarylrest ist, der gegebenenfalls ein- oder mehrfach mit R⁴ und/oder R³ substituiert ist,
R¹ ein Wasserstoff oder ein CH₃-Rest ist,
R² ein Wasserstoff, Fluor oder CH₃-Rest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
sowie deren Isomere, Diastereomere, Enantiomere und Salze bzw. Cyclodextrinclathrate, für die Herstellung eines Arzneimittels zur Fertilitätskontrolle.

7. Verbindungen gemäß Anspruch 1 - 6,
wobei
X eine -CH=CH- Gruppe, und
Y eine -(CH₂)₂- Gruppe bedeuten kann, wenn
A ein Phenylrest ist, der gegebenenfalls ein- oder zweifach mit R⁴ und/oder R³ substituiert ist,
R¹ ein Wasserstoff oder ein CH₃-Rest ist,
R² ein Wasserstoff, Fluor oder CH₃-Rest ist,
R³ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
R⁴ ein Wasserstoff, Halogen, Amino, CF₃, Hydroxy, ein C₁-C₄-Alkyl, ein C₁-C₄-Alkoxy oder N-(C₁-C₄-Alkyl)₂ ist,
oder wobei
X eine-CH₂-CH₂- Gruppe, und
Y eine -(CH₂)₂- Gruppe bedeuten kann, wenn
A ein Phenyl- oder Pyridylrest ist, der gegebenenfalls ein- oder mehrfach mit R⁴ und/oder R³ substituiert ist,
R¹ ein Wasserstoff oder ein CH₃-Rest ist,
R² ein Wasserstoff, Fluor oder CH₃-Rest ist,
R³ ein Wasserstoff, Halogen, C₁-C₄-Alkyl oder C₁-C₄-Alkoxy ist,
R⁴ ein Wasserstoff, Halogen, C₁-C₄-Alkyl oder C₁-C₄-Alkoxy ist,
bedeuten, sowie deren Isomere, Diastereomere, Enantiomere und Salze bzw. Cyclodextrinclathrate, für die Herstellung eines Arzneimittels zur Fertilitätskontrolle.

8. Verbindungen gemäß den vorangegangenen Ansprüchen ausgewählt, aus einer Gruppe, die die folgenden Verbindungen enthält:
1. E)-N-[2-(7-Fluoro-2,4-dimethyl-1 H-indol-3-yl)-ethyl]-3-(4-methoxy-phenyl)-acrylamid
2. (E)-3-(4-Chloro-phenyl)-N-[2-(7-fluoro-2,4-dimethyl-1H-indol-3-yl)-ethyl]-acrylamid
3. (E)-N-[2-(7-Fluoro-2,4-dimethyl-1 H-indol-3-yl)-ethyl]-3-(3-methoxy-phenyl)-acrylamid
4. 3-(3-Ethoxy-phenyl)-N-[2-(7-fluoro-2,4-dimethyl-1 H-indol-3-yl)-ethyl]-propionamid
5. N-[2-(7-Fluoro-2,4-dimethyl-1H-indol-3-yl)-ethyl]-3-pyridin-3-yl-propionamid
6. N-[2-(7-Fluoro-2,4-dimethyl-1 H-indol-3-yl)-ethyl]-3-(4-fluoro-phenyl)-propionamid
7. (E)-N-[2-(7-Fluor-2,4-dimethyl-1H-indol-3-yl)-ethyl]-3-p-tolyl-acrylamid
8. (E)-3-(4-Brom-phenyl)-N-[2-(7-fluor-2,4-dimethyl-1 H-indol-3-yl)-ethyl]-acrylamid
9. (E)-N-[2-(4,7-Difluor-2-methyl-1H-indol-3-yl)-ethyl]-3-(4-methoxy-phenyl)-acrylamid
10. (E)-3-(3,4-Dimethoxy-phenyl)-N-[2-(7-fluor-2,4-dimethyl-1 H-indol-3-yl)-ethyl]-acrylamid
11. (E)-N-[2-(7-Fluor-2,4-dimethyl-1H-indol-3-yl)-ethyl]-3-(2-methoxy-phenyl)-acrylamid
12. (E)-N-[2-(7-Fluor-2,4-dimethyl-1 H-indol-3-yl)-ethyl]-3-phenyl-acrylamid
13. (E)-N-[2-(7-Fluor-2,4-dimethyl-1 H-indol-3-yl)-ethyl]-3-m-tolyl-acrylamid
14. (E)-N-[2-(7-Fluor-2,4-dimethyl-1H-indol-3-yl)-ethyl]-3-o-tolyl-acrylamid
15. (E)-N-[2-(7-Fluor-2,4-dimethyl-1 H-indol-3-yl)-ethyl]-3-(4-hydroxy-phenyl)-acrylamid
16. (E)-N-[2-(7-Fluor-2,4-dimethyl-1 H-indol-3-yl)-ethyl]-3-(3-fluoro-4-methoxyphenyl)-acrylamid
17. (E)-3-(4-Dimethylamino-phenyl)-N-[2-(7-fluor-2,4-dimethyl-1 H-indol-3-yl)-ethyl]-acrylamid
18. (E)-3-(2,4-Difluor-phenyl)-N-[2-(7-fluor-2,4-dimethyl-1 H-indol-3-yl)-ethyl]-acrylamid
19. (E)-3-(3,5-Dimethyl-phenyl)-N-[2-(7-fluor-2,4-dimethyl-1 H-indol-3-yl)-ethyl]-acrylamid
20. (E)-3-(3,5-Difluor-phenyl)-N-[2-(7-fluor-2,4-dimethyl-1 H-indol-3-yl)-ethyl]-acrylamid
21. (E)-3-(2-Chlor-phenyl)-N-[2-(7-fluor-2,4-dimethyl-1 H-indol-3-yl)-ethyl]-acrylamid
22. (E)-N-[2-(7-Fluor-2,4-dimethyl-1 H-indol-3-yl)-ethyl]-3-(3-hydroxy-phenyl)-acrylamid
23. (E)-3-(3,4-Difluor-phenyl)-N-[2-(7-fluor-2,4-dimethyl-1H-indol-3-yl)-ethyl]-acrylamid
24. (E)-N-[2-(7-Fluor-2,4-dimethyl-1 H-indol-3-yl)-ethyl]-3-(2-fluoro-phenyl)-acrylamid

9. Verwendung der Verbindungen gemäß den Ansprüchen 1 - 8 zur Herstellung von Arzneimitteln, die mindestens eine der Verbindungen der Formel I enthalten.

10. Arzneimittel gemäß Anspruch 9 mit geeigneten Formulierungs- und Trägerstoffen.

11. Verwendung der Arzneimittel gemäß Anspruch 9 und 10, **dadurch gekennzeichnet, dass** das Arzneimittel zur Behandlung und Prophylaxe von Erkrankungen verwendet wird.

12. Verwendung gemäß Anspruch 10, zur Behandlung und Prophylaxe von Erkrankungen, die im Zusammenhang mit dem EP₂-Rezeptor stehen.

13. Verwendung gemäß Anspruch 10 zur Behandlung und Prophylaxe von Fertilitätsstörungen.

14. Verwendung gemäß Anspruch 10 zur Behandlung und Prophylaxe von Menstruationsbeschwerden.

15. Verwendung gemäß Anspruch 10 zur Behandlung und Prophylaxe von Endometriose.

16. Verwendung der Verbindungen gemäß Anspruch 1-8 zur Modulation des EP₂ Rezeptors.

17. Verwendung gemäß Anspruch 10 zur Behandlung und Prophylaxe von Schmerz.

18. Verwendung der Verbindungen gemäß Anspruch 1 - 8 und der Arzneimittel gemäß Anspruch 9 zur Fertilitätskontrolle/Kontrazeption.

19. Verwendung gemäß Anspruch 10 zur Behandlung und Prophylaxe von Osteoporose.

20. Verwendung gemäß Anspruch 10 zur Behandlung und Prophylaxe von Krebs.

21. Verwendung der Verbindungen der allgemeinen Formel I, gemäß den Ansprüchen 1 - 8, in Form eines pharmazeutischen Präparates für die enterale, parenterale, vaginale und orale Applikation.
